# EUROPEAN PATENT SPECIFICATION

(11) **EP 2 632 430 B1**
(45) Date of publication and mention of the grant of the patent: **06.06.2018**
(21) Application number: 11797111.9
(22) Date of filing: 28.10.2011
(51) Int. Cl.: A61K 9/00, A61K 31/714, A61K 47/10, A61K 47/22

(54) **NASAL COMPOSITIONS OF VITAMIN B12**
ZUSAMMENSETZUNGEN ZUR NASALEN VERABREICHUNG VON VITAMIN B12
COMPOSITIONS NASALES DE LA VITAMINE B12

(30) Priority: 29.10.2010 IN 2169MU2010
(43) Date of publication of application: 04.09.2013
(73) Proprietor: Troikaa Pharmaceuticals Ltd, Ahmedabad 380 054 GUJ (IN)
(72) Inventor: PATEL, Ketan, R., Ahmedabad 380 054 Gujarat (IN); PATEL, Milan, R., Ahmedabad 380 054 Gujarat (IN); SHAH, Prakashchandra, J., Ahmedabad 380 054 Gujarat (IN)
(74) Representative: Høiberg P/S
(86) International application number: PCT/IB2011/002546
(87) International publication number: WO 2012/056299

(56) References cited:
- DE-A1- 3 337 304
- ES-A1- 2 074 396
- US-A- 4 724 231
- US-A1- 2009 012 039
- US-B1- 7 229 636

## Description

### FIELD OF THE INVENTION:

The present invention relates to intranasal compositions of the vitamin B-12 derivatives methylcobalamin and cyanocobalamin.

### BACKGROUND OF THE INVENTION:

Vitamin B-12 deficiency is very common among population. Large surveys in the United States and the United Kingdom disclosed that about 6% of those aged above or equal to 60 years are vitamin B-12 deficient. Moreover, in developing countries like India this deficiency is much more common, starting in early life and persisting across the life span. A study of 441 middle-aged men in Pune (India) revealed that 67% of the men had low vitamin B-12 concentration (<150 pmol/L). Of the urban middle class, 81% had low vitamin B-12 concentration and vegetarians had 4.4 times higher risk of low vitamin B-12 concentrations.

The main causes of B-12 deficiency include lack of intrinsic factors and other intestinal factors (e.g. malabsorption), rare genetic disorders, and inadequate intake. Therefore, it is necessary to overcome the deficiency of B-12 by supplementing with Cyanocobalamin, Hydroxocobalamin or Methylcobalamin through various routes such as Parenteral, oral etc.

Oral therapy is not suitable for all patients e.g. patients with lack of intrinsic factors, conditions associated with gastric atrophy, infestation with fish tape worm etc. For such patients nasal administration would be more appropriate. Delivery of drugs via the intra-nasal route also offers effective delivery of a drug to the systemic circulation resulting in better therapeutic effectiveness. It is also known that for improved systemic delivery of a drug through intra-nasal route it is essential to have the drug solubilized. Generally a highly soluble drug is considered as a good candidate for such therapies.

Methylcobalamin is based on a corrin ring and has the molecular formula C63H91CoN13O14P. Compounds belonging to the class of Vitamin B-12 variants also include Cyanocobalamin and Hydroxycobalamin. Methylcobalamin has much lower solubility in water as compared to Cyanocobalamin or Hydroxycobalamin. In water, Hydroxycobalamin has the highest solubility whereas, Methylcobalamin exhibits the lowest solubility.

Methylcobalamin is also known to be highly susceptible to degradation and therefore the least stable among all the derivatives of Vitamin B-12. In view of the rapid degradation of Methylcobalamin, it is a usual practice to add high overages ranging from 50% to 100 % in liquid formulations of Methylcobalamin (e.g. Injections), to compensate for the loss due to degradation on storage. Overages in such a high concentration are not desirable as they result in fairly high amounts of degradation product as the life of the product steps-up.

Use of Cyanocobalamin in the dose of at least 500 mcg/0.1ml a week by intranasal administration is known. Use of Methylcobalamin for the treatment of Vitamin B12 deficiency as well as for the diseases like ADHD is also known. However, it is less preferred over other Cobalamin derivatives and not used in commercially available intra-nasal formulations due to its inherent problems of low solubility and high instability.

US Patent No. 4,724,231 describes an intranasal preparation of Cyanocobalamin dissolved in water presented in a concentration of 500 mcg/0.1 ml. Further the compositions disclosed in the said patent are in the form of gels, creams, ointments, having high viscosities in the range of 2,500-10,000 CPS (mPas). The inventors have chosen the high viscosity range to increase the residence time of the composition with the nasal mucosa. Such highly viscous gels are difficult to administer with accurate dosing and causes discomfort to the patient throughout the treatment span.

US Patent Application 20090012039 discloses a method of use of an intranasal Methylcobalamin preparation for the treatment of attention deficit disorder. The said patent application discloses nasal administration compositions, which can be prepared by dissolving, dispersing, mixing or incorporating Methylcobalamin (with or without folinic acid) and optional pharmaceutical excipients, such as water, saline, aqueous dextrose, glycerol, ethanol, and the like, to form a solution or suspension. It is mentioned that the composition to be nasally administered may also contain minor amounts of nontoxic auxiliary substances such as wetting or emulsifying agents, pH buffering agents and the like, for example, sodium acetate, sorbitan mono-laurate, triethanolamine sodium acetate, triethanolamine oleate, potassium sorbate, glycerin, lecithin, etc if required.

US Patent 7229636 discloses low viscosity aqueous formulations of Cyanocobalamin for intranasal delivery having pH in the range of 4 to 6 with 4.5 to 5.5 being the most preferred and exemplified range. The patent addresses the problems of unpleasant consistency of the marketed intranasal gel formulations (NASCOBAL) to provide acceptable low viscosity intra-nasal spray formulations of Cyanocobalamin.. Cynocobalamin has been preferred over Methylcobalamin due to its higher stability and the ease of formulation development.

Though the prior art discloses intranasal formulations of Cynocobalamin and some approaches to the use of Methylcobalamin, issues with regard to the low solubility, instability of Methylcobalamin, and therefore their impact on the bioavailability for optimized therapeutic effectiveness remain unaddressed. Further the optimized viscosity and pH of formulations for effective intranasal delivery remains a challenge and hence therapeutically effective commercial formulations of Methylcobalamin are not available in the market. Further it would be desirable to provide formulations to enhance the bio-availability of formulations of the family of Vitamin B-12 derivatives to achieve improved therapeutic efficacy.

The present invention provides novel and stable intranasal formulations of Methylcobalamin with optimized concentrations of the drug in its solubilized form and viscosity of the formulation to achieve the desired bioavailability for therapeutic effectiveness. It also provides novel formulations of other derivatives of Vitamin B12, viz. cyanocobalamin, with significantly higher bioavailability as compared to prior art preparations.

### OBJECTS OF THE INVENTION:

The main object of the invention is to provide intranasal compositions of the class of vitamin B-12 derivatives such as Methylcobalamin, cynocobalamin and Hydroxocobalamin and process for preparation thereof

Another object of the invention is to provide stable, high concentration formulations for intranasal applications with enhanced bio-availability of Methylcobalamin to achieve improved therapeutic efficacy by use of targeted penetration enhancers.

Another object of the invention is to provide high concentration formulations of other derivatives of Vitamin B12 eg. Cyanocobalamin, which have significantly higher bioavailability as compared to the preparations in prior art.

Another object of the invention is to provide stable, high concentration, clear transparent solutions of Methylcobalamin in water by the use of suitable co-solvents/solubilizer or mixtures thereof, with or without penetration enhancers to maximize bio-availability of the drug upon intra-nasal administration.

Another object of the invention is to provide stable, high concentration, clear transparent solutions of Methylcobalamin in water by the use of suitable co-solvents/ solubilisers or mixtures thereof with or without penetration enhancers, wherein the quantum of the co-solvents /solubilisers or mixtures thereof is selected in a manner such that the formulation results in enhanced solubility without significantly increasing the viscosity of the intranasal formulation.

Another object of the invention is to provide the said stable, high concentration, clear transparent solutions of Methylcobalamin in concentrations of Methylcobalamin from about 500 mcg/0.1 ml to about 1500 mcg/0.1 ml.

Another object of the invention is to provide the said stable high concentration, clear transparent solutions of Methylcobalamin in water, having a pH suitable for systemic intranasal drug delivery of Methylcobalamin ranging between about 5.0 to about 7.0.

Another object of the invention is to provide the said stable high concentration, clear transparent solutions of Vitamin B12 derivatives in water, having viscosity ranging from about 1 to 200 CPS (mPas) suitable for systemic intranasal drug delivery.

Another object of the invention is to provide said stable high concentration, clear transparent solutions of Methylcobalamin in water, optionally having suitable quantity of a mucoadhesive agent or mixtures thereof which are suitable for systemic intranasal drug delivery.

Another object of the invention is to provide the said high concentration, clear transparent solutions of Methylcobalamin in water, with or without penetration enhancers such as bile salts, Vitamin E TPGS, Alkyl Maltosides or mixtures thereof, which are suitable for systemic intranasal drug delivery.

Another object of the invention is to provide the said stable high concentration, clear transparent solutions of Methylcobalamin in water, in combination with suitable quantity of chelating agents such as Disodium EDTA, which are suitable for systemic intranasal drug delivery.

Another object of the invention is to provide the said stable high concentration clear transparent solutions of Methylcobalamin in water, in combination with suitable preservatives and antioxidants.

### DETAILED DESCRIPTION OF THE INVENTION:

The present invention provides a stable intranasal aqueous composition comprising methylcobalamin in concentration from 500mcg/0. 1ml to 1500mcg/0.1ml, co-solvents/solubilisers or mixtures thereof in water, wherein the cosolvent/solubiliser is selected from glycofurol, propylene glycol, polyethylene glycols, or mixtures thereof, optionally a penetration enhancer, and wherein the pH of the composition is 6 to 6.5 and viscosity of 1 to 200 mPas. Further, the present invention provides a stable intranasal aqueous composition comprising cyanocobalamin in concentration from 500mcg/0.1ml to 1500mcg/0.1ml, co-solvents/solubilisers or mixtures thereof in water, and at least one penetration enhancer, wherein the cosolvent/solubiliser is selected from glycofurol, propylene glycol, polyethylene glycols, or mixtures thereof, wherein the penetration enhancer is selected from bile salts such as sodium glycocholate and wherein the pH of the composition is 4 to 6 and viscosity of 1 to 200 mPas. Methylcobalamin has much lower solubility in water as compared to Cyanocobalamin or Hydroxocobalamin. To provide optimum therapeutic benefit by the nasal route, it is necessary to solubilize of Methylcobalamin such that the solutions have concentration ranging from about 500 mcg per 0.1 ml to about 1500 mcg per 0.1 ml.. We have surprisingly found that solubilization can be achieved in the desired concentration range to produce solutions of controlled viscosities, by the judiciously formulating a solvent system using co-solvents/solubilisers viz. glycofurol, propylene glycol, polyethylene glycols or mixtures thereof individually or in combination with water, wherein the amount of the co-solvents/solubilisers is from 1 to 35%w/v. Moreover, the formulations have been optimized in terms of their viscosity and pH to ensure maximization of intranasal absorption of the drug to achieve effective therapeutic efficacy and bioavailability with improved patient compliance.

In addition to the solvent system, the formulation includes other pharmaceutically acceptable excipients selected from but not limited to a group of excipients comprising humectants such glycerin, and / or preservatives and/or antioxidants/ or penetration enhancers and/or mucoadhesives.

The preservatives of the present invention are selected from Benzalkonium chloride, methyl paraben, ethyl paraben, propyl paraben, benzoic acid, benzyl alcohol, bronopol, butylated, hydroxyl anisole, butylated hydroxyl toluene, chlorocresol, or Isopropyl alcohol or combination thereof.

The penetration enhancers of the present invention can be selected from bile salts, Vitamin E TPGS, Alkyl Maltosides, non-ionic,' anionic or amphoteric surfactants having HLB value 8-14 or combination thereof. The non-limiting examples of such penetration enhancers are sodium glycocholate, sodium taurocholate, dodecyl maltosides, tridecyl maltoside or tetradecyl maltosides, or any combination thereof. Penetration enhancers are used upto 2.5 % by weight of the compositions .

The mucoadhesives are selected from synthetic polymers such as Cellulose derivatives (methylcellulose, ethylcellulose, hydroxy-ethylcellulose, Hydroxyl propyl cellulose, hydroxy propyl methylcellulose, sodium carboxy methylcellulose; Poly (acrylic acid) polymers (carbomers or carbopols, polycarbophil) Such as carbopol 934; Poly (hydroxyethyl methylacrylate); Poly (ethylene oxide); Poly (vinyl pyrrolidone); Poly (vinyl alcohol). The muco-adhesives are further selected from Natural polymers such as Tragacanth, Sodium alginate, Karaya gum, Guar gum, Xanthan gum, Lectin, Soluble starch, Gelatin, Pectin and Chitosan and are used upto 5% by weight of the composition.

The humectants are selected from glycerine, sodium lactate, sorbitol, Xylitol, butylenes glycol.

We have surprisingly found that it is possible to achieve the desired viscosity of about 1 to 200 CPS (mPas) by a judicious combination of the ingredients especially the solvent system and yet provide significantly enhanced transnasal absorption of Methylcobalamin. The formulations are presented the form of a solution, drops or spray packs, in multi-dose containers, for delivery of predetermined volumes. It is important note that despite the lower viscosity of the formulations of the present invention, they do not have compromised trans-nasal absorption of the drug. Prior art teaches that formulations ought to have high viscosity (US Patent 4724231 recommending viscosities of 2500 to 10000 CPS (mPas)) to ensure the desired residence time on the nasal mucosa. The present invention is a distinct deviation from the teachings of the prior art and yet achieves a perfect balance between the viscosity, sprayability, absorption and enhanced bioavailability.

The formulations of present invention optionally comprise additional pharmaceutically acceptable excepients that are used for further augmenting the stability of the formulations. Non-limiting examples of such excepients are chelating agents such as Sodium EDTA and/or anti-oxidants which do not irritate the nasal mucosa. Non-limiting example of such anti-oxidants is ascorbic acid.

The formulations of methylcobalamin nasal solutions disclosed herein exhibit desirable stability thereby enabling lower overages.

Merely achieving solubilization is not a sufficient condition to ensure maximum absorption of the drug such as Methylcobalamin. Parameters such as viscosity of the formulation, stability of the drug with respect to shelf life also need to be optimized to ensure the required transnasal absorption and bioavailability for therapeutic effectiveness.

A range of solutions having pH in the range of about 3.75 to 7 were prepared. Suitable buffer systems such as citrate buffer may be used to achieve the desired pH. It was then necessary to determine the impact of pH on intranasal absorption of Methylcobalamin.

In order to evaluate the impact of pH on intranasal absorption of Methylcobalamin, solutions of Methylcobalamin were prepared and bio-availability studies were conducted for the formulations exhibiting pH 3.75, 5, and 6.25. We have surprisingly found that the pH of above 6 is most appropriate to achieve the desired intra-nasal absorption of Methylcobalamin. The results are given in Tables 1 & 2 below.

**Table 1**

| **Ingredients** | **Test A** | **Test B** | **Test C** |
|---|---|---|---|
| Methylcobalamin | 500 mcg | 500 mcg | 500 mcg |
| Benzalkonium Chloride | 0.02 mg | 0.02 mg | 0.02 mg |
| Glycerin | 2.23 mg | 2.23 mg | 2.23 mg |
| Glycofurol | 1.00 mg | 1.00 mg | 1.00 mg |
| Sodium citrate di-hydrate | 0.32 mg | 0.32 mg | 0.32 mg |
| Citrate Acid Anhydrous (5%w/v) | q.s. | q.s. | q.s. |
| Water | q.s. to 0.1 ml | q.s. to 0.1 ml | q.s. to 0.1 ml |
| pH Adjusted to | 6.25 | 5.00 | 3.75 |

**Results:** AUC0-t (pg.hr/ml) for Mecobalamin with the dose of 1500 mcg and adjusted dose of 500 mcg with baseline correction is presented below.

**Table 2**

| **Test** | **AUC0-t (pg.hr/ml) (For 1500 mcg dose)** | **AUC0-t (pg.hr/ml) (For dose adjusted to 500 mcg)** |
|---|---|---|
| Test A | 8374.328 | 2791.44 |
| Test B | 5120.904 | 1702.18 |
| Test C | 4044.998 | 1348.33 |

### Process for the preparation of the compositions:

The entire preparation was carried out under nitrogen flushing under sodium vapour lamp. The dissolved oxygen levels were kept to a minimum feasible, preferably around 1 to 1.5 ppm. The process comprises steps of
i) Adding Glycofurol to 80 % v/v of water for the batch;
ii) Dissolving Benzalkonium chloride and glycerin with stirring;
iii) Buffering the solution using citrate buffer;
iv) Adding Methylcobalamin with stirring till dissolution;
v) Adjusting pH to the requisite value by addition of acid/alkali
vi) Making up the volume of the solution to obtain the desired concentration of 500mcg per 0.1 ml of the nasal spray solution.

The compositions of the present invention would typically comprise:
the drug 500mcg/0.1ml to 1500mcg/0.1ml; Solvents ( total ) 1-35 %; Humectant 0-5 %, Mucoadhesive upto 5 %; Penetration enhancers upto 2.5% ; Preservatives upto 4 %. As further described in the specification, the compositions would further contain the buffering ingredients to adjust the pH to the requisite value, depending on the drug used.

### STUDIES ON BIOAVAILABILITY OF FORMULATIONS

A bioavailability study of the nasal sprays prepared as described above, containing 500 mcg/ 0.1 mL of Methylcobalamin was conducted in healthy human subjects under fasting condition. The design of the study was Open Label, Randomized, Three period, Three Treatment, Three sequence, Single Dose, 3-way Crossover Comparative bioavailability study under fasting condition in 6 healthy adult human subjects.
**Methods:** The study was carried out to determine the comparative bioavailability of the three Test products by evaluating of the Pharmacokinetic parameters, Cmax, AUC0-t, AUC 0-inf. under fasting conditions in 6 healthy, adult, human subjects in a randomized crossover study. All the subjects provided written informed consent to participate in the study prior to enrolment and were free to withdraw at any time during the study. The study was approved by the institutional ethics committee and was conducted in accordance with Good Clinical Practice and the Declaration of Helsinki.
**Study products:** (Formula disclosed in Table 1)
**Test Product (A):** Mecobalamin Nasal Spray 500 mcg/ 0.1 ml
**Test Product (B):** Mecobalamin Nasal Spray 500 mcg/ 0.1 ml
**Test Product (C):** Mecobalamin Nasal Spray 500 mcg/ 0.1 ml
**Pre-study evaluation:** Subjects were screened for demographic data, medical history, physical examination, 12 lead ECG, Hemogram, biochemistry, serology, urinalysis and urine screen for drug analysis

### Drug Administration:

Subjects received each treatment (Mecobalamin Nasal Spray) in a crossover fashion according to the Formulation Randomization Schedule with a wash out period of at least 7 days between each period.

After overnight fast of 10 hrs, subjects were administered the scheduled dose of the Test Formulation (A) or Test Formulation (B) or Test Formulation (C) which were administered by nasal route, under the supervision of a medical personnel.

### Priming (Activation) of nasal spray:

Before the administration of first dose, the pump was primed. To prime the pump, nozzle was placed between the first and second finger with the thumb on the bottom of the bottle. The unit was pumped firmly and quickly until the first appearance of spray. The pump was primed for an additional 2 times and the nasal spray was ready for use.

### Administration Procedure:

Subjects were asked to tilt the head slightly backward for ease of administration of nasal spray. Then it was sprayed three times deep into the nostril. Subjects were asked to sit in same position for two minutes so that the dose doesn't trickle out.
**Monitoring of subject during study:** Physical Examination, Vital Sign monitoring and Well Being was monitored at 0.00 hour (pre-dose), 2.00, 5.00, 12.00, 24.00 and 36.00 hours (Post-dose) ± 30 minutes, and as and when required.
**Post Study assessment:** 12 lead ECG, Hemogram, biochemistry and urinalysis was done after last blood sample collection of period-Ill or on discontinuation/withdrawal of subject from the study.
**Housing:** From approximately 12 hours prior to drug administration and until 36 hours after drug administration.
**Meals:** Standardized meals were provided to the subjects at 4.00, 8.00, 12.00, 25, 29 and 33 hours post-dose in each study period.

### Sample Collection:

Total No. Of Blood Samples: Twenty (20) per period.
Sampling Hours: -10.00, -06.00, 0.00 hrs (pre-application samples for baseline), 0.50, 0.75, 1.00, 1.25, 1.50, 1.75, 2.00, 2.50, 3.00, 4.00, 5.00, 6.00, 8.00, 12.00, 18.00, 24.00 and 36.00 hrs (post-application). Blood samples were collected in amber color/ covered with aluminum foil vacutainer.
**Total Blood Loss:** Blood sample of 5.0 mL was collected and 0.1 mL was discarded before each sample collection that represents 306 ml. A total of approximately 326 mL (including 20 mL for pre and post study Clinical Laboratory tests) of blood was drawn from each subject, for all the periods.
**Sample processing:** Blood samples were allowed to clot for 1 hour at room temperature and then centrifuged at 4000 ± 300 rpm for 10 min. The serum was separated and rapidly transferred to appropriate size, amber color polypropylene screw top (previously labeled with study code and sample code) vials, in duplicate. All the samples were stored in a deep freezer maintained at -20°C ± 5°C.

### Pharmacokinetic Parameters:

Primary parameters: Cmax, AUC0-t, AUC0-inf
Secondary parameters: t½, ke, Tmax

### Analytical Method:

Serum concentration of Mecobalamin was measured by pre-validated analytical method.

### Statistical Evaluation

ANOVA, two one sided tests for comparing and ratio analysis for untransformed and log transformed Pharmacokinetic parameters C max, AUC0-t, AUC0-inf were performed. As all the parameters were indicating similar conclusions, AUC0-t (pg.hr/ml), the most relevant parameter for therapeutic benefit is provided in the results for the exemplified formulations.

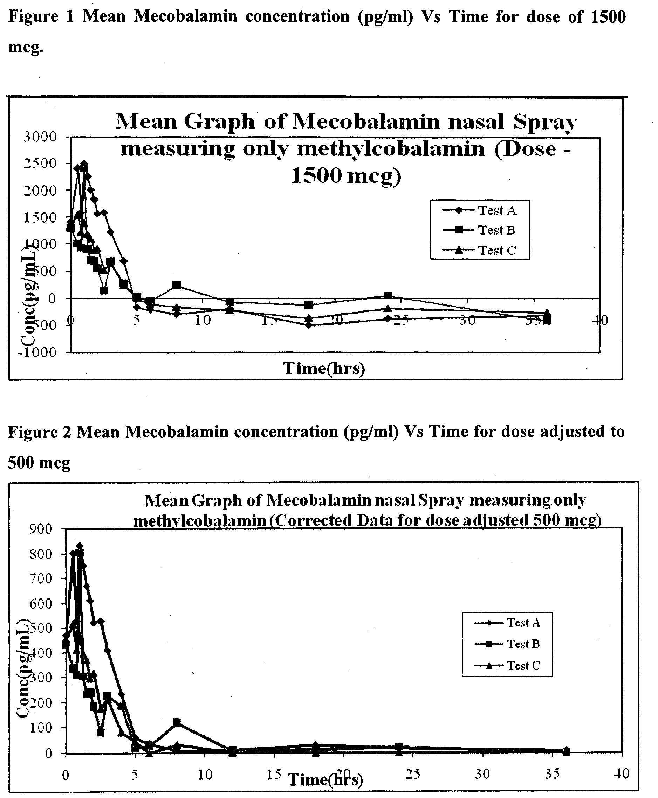

This study conducted for the dose of 1500 mcg Methylcobalamin and the statistical analysis was done for the dose of 1500 mcg as well as for the adjusted dose of 500 mcg. The mean plasma concentration-time profile for the dose of 1500 mcg is shown in the figure 1 and the mean plasma concentration-time profile for corrected data for single dose of 500 mcg is shown in the figure 2. Mean area under curve (AUC0-t) for the dose of 1500 mcg and for 500 mcg of test formulations are listed in Table 2.
**Results:** AUC0-t (pg.hr/ml) for Mecobalamin with the dose of 1500 mcg and adjusted dose of 500 mcg with baseline correction is presented below.

**Table 3**

| **Test** | **AUC0-t (pg.hr/ml) (For 1500 mcg dose)** | **AUC0-t (pg.hr/ml) (For dose adjusted to 500 mcg)** |
|---|---|---|
| Test A | 8374.328 | 2791.44 |
| Test B | 5120.904 | 1702.18 |
| Test C | 4044.998 | 1348.33 |

Results of the bioavailability studies of all the three formulations shows that the test A was found to provide the maximum transnasal delivery of Methylcobalamin. It is noteworthy that the availability of Methylcobalamin increased drastically by increasing the pH from 3.75 to 6.25. The graphs for the dose of 1500 mcg as well as the one for dose adjusted to 500 mcg are provided in Figure 1 and Figure 2.

Surprisingly, the pH range for favourable absorption of Methylcobalamin was found to be 5-7 with the most preferred pH being 5.5 to 7 with pH of 6.25 being the most appropriate for the highest absorption. It may be noted that pH values higher than 7 are not preferred as high pH facilitates growth of pathogenic bacteria in the nasal passage.

It was further surprisingly found that addition of penetration enhancers such as bile salts, Vitamin E TPGS (d-alpha-tocopheryl polyethylene glycol 1000 succinate), or Alkyl maltosides such as dodecyl maltosides, tridecyl maltoside or tetradecyl maltoside or any combination thereof, significantly impact the absorption and bioavailability of Methylcobalamin when the formulation is delivered nasally.

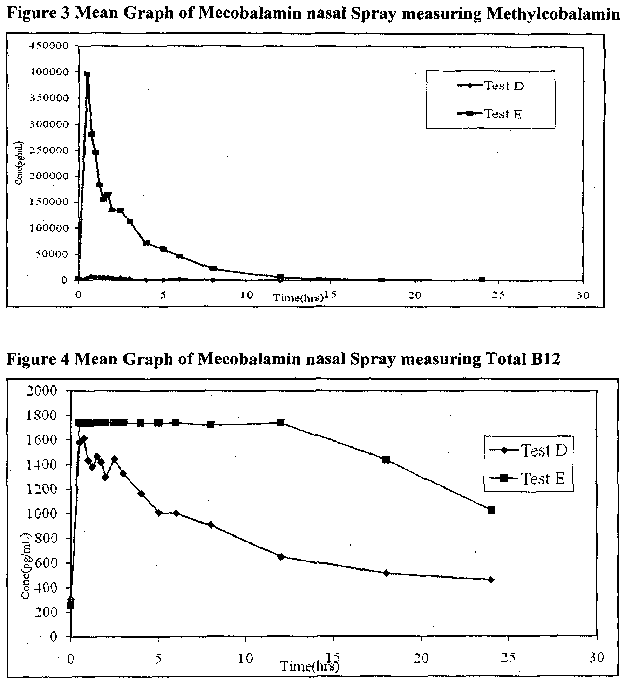

Accordingly, a formulation of Methylcobalamin without the penetration enhancer (Test D) was compared with a formulation of Methylcobalamin with 2% penetration enhancer (Test E) to assess the effect of the penetration enhancer on the intra-nasal absorption of Methylcobalamin. The pH of these formulations was adjusted to 6.25 ± 0.25. The composition of these test formulations are as disclosed in the table below. Please also refer to Figure 3 and Figure 4.

**Table 4**

| **Ingredients** | **Test D** | **Test E** |
|---|---|---|
| Methy lcobalamin | 500 mcg | 500 mcg |
| Benzalkonium Chloride | 0.02 mg | 0.02 mg |
| Glycerin | 2.23 mg | 2.23 mg |
| Glycofurol | 1.00 mg | 1.00 mg |
| Sodium Glycocholate | - | 2.00 mg |
| Sodium citrate di-hydrate | 0.32 mg | 0.32 mg |
| Citrate Acid Anhydrous (5%w/v) | q.s. | q.s. |
| Water | q.s. to 0.1 ml | q.s. to 0.1 ml |
| pH Adjusted to | 6.23 | 6.28 |

**Table 5**

| **Test** | **Component Measured** | **AUC0-t (pg.hr/ml) (Baseline corrected)** |
|---|---|---|
| Test D | Methylcobalamin | 22323.09 |
| | Total B12 | 18651.79 |
| Test E | Methylcobalamin | 897877.47 |
| | Total B12 | 37399.75 |

The results surprisingly show that the trans-nasal penetration of Methylcobalamin was increased significantly in the presence of a small amount of the penetration enhancer. This was further confirmed by the results of the study measuring the plasma concentration of Total vitamin B12. Since higher concentrations of sodium glycocholate were found to cause nasal irritation, the concentration of sodium glycocholate was preferably adjusted to be not more than 2.5% w/v of the formulations.

Formulations similar to Test E with varying concentration of sodium glychocolate viz. 2% w/v, 1.5% w/v, and 1 % w/v were prepared and evaluated for the trans-nasal absorption of Methylcobalamin through a three way bio-equivalence study. The results of the study indicating the total vitamin B12 levels are as shown below.

**Table 6**

| **Test** | **Component Measured** | **AUC0-t (pg.hr/ml) (Baseline corrected)** |
|---|---|---|
| Formulation with 2% Sodium Glycocholate | Total B12 | 43446.58 |
| Formulation with 1.5% Sodium Glycocholate | Total B12 | 27748.35 |
| Formulation with 1.% Sodium Glycocholate | Total B12 | 208134.59 |

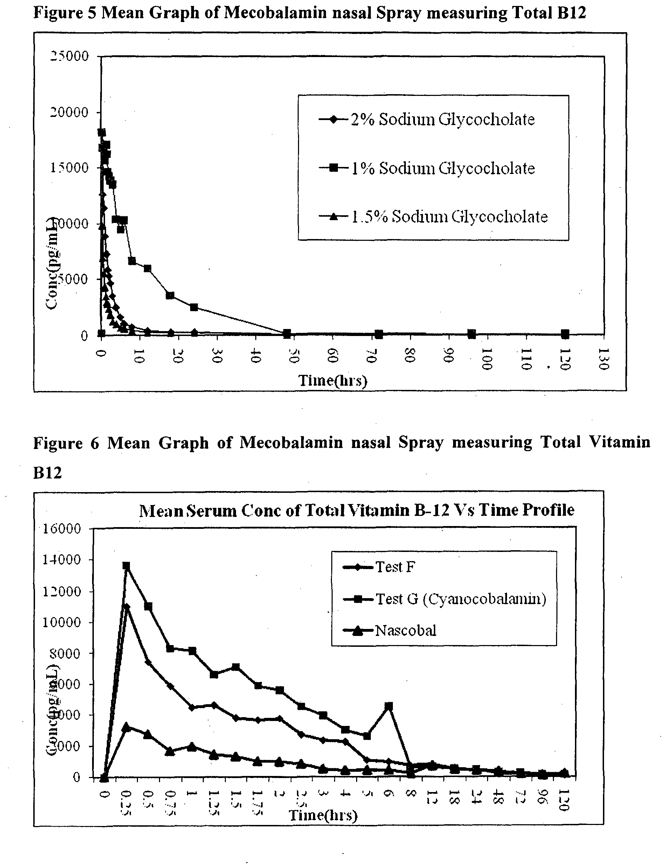

Results of the above study, as also shown in Figure 5, clearly demonstrate that incorporation of 1% w/v sodium glycocholate results in higher absorption of methylcobalamin as compared to 2% w/v sodium glycocholate. In other words, the absorption increases when amount of penetration is reduced from 2% w/v to 1% w/v. Accordingly, decreasing the amount of the penetration enhancer resulted in higher absorption.

Another set of experiments were conducted to access the role of penetration enhancer such as sodium glycocholate in formulations containing Cyanocobalamin or Hydroxocobalamin.

A three way bio-equivalence study was conducted to evaluate the effect of sodium glycocholate on intra-nasal absorption of Cyanocobalamin as well as to compare the Methylcobalamin levels produced by respective formulations. The formulations subjected to the bio-equivalence study comprised Cyanocobalamin formulations prepared incorporating sodium glycocholate in accordance with the present invention (Test G), marketed intranasal formulation of Cyanocobalamin spray (Nascobal-USA) and Methylcobalamin formulation of present invention (Test F). The formulas of Test F and Test G are disclosed in the table below.

**Table 7**

| **Ingredients** | **Test F** | **Test G** |
|---|---|---|
| Methylcobalamin | 500 | -- |
| Cyanocobalamin | -- | 500 |
| Benzalkonium Chloride | 0.02 mg | 0.02 mg |
| Glycerin | 2.23 mg | 2.23 mg |
| Glycofurol | 1.00 mg | 1.00 mg |
| Sodium Glycocholate | 1.00 mg | 1.00 mg |
| Sodium citrate di-hydrate | 0.32 mg | 0.32 mg |
| Citric Acid Anhydrous (5%w/v) | q.s. | q.s. |
| Water | q.s. to 0.1 ml | q.s. to 0.1 ml |

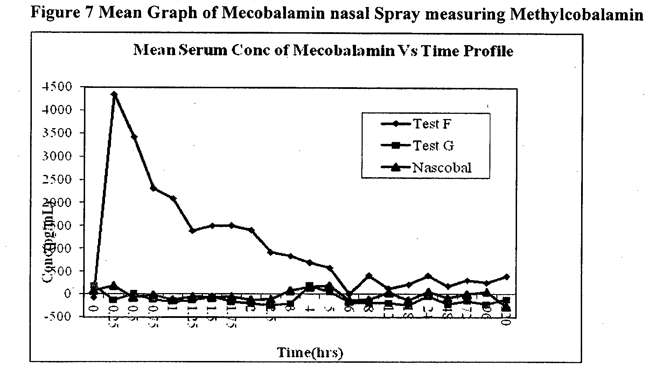

The plasma concentration of Methylcobalamin as well as total Vitamin B12 was measured to assess the effect of administering Methylcobalamin over Cyanocobalamin for intra-nasal formulations. Nascobal® Nasal Spray is a solution of Cyanocobalamin in the form of a spray for the delivery to the nasal mucosa. Each bottle of Nascobal Nasal Spray contained 2.3 ml of a 500 mcg / 0.1 ml solution of Cyanocobalamin with sodium citrate, citric acid, glycerin and Benzalkonium chloride in purified water. The marketed formulation of Cyanocobalamin (Nascobal) was assayed and then the Test F and G formulations were prepared in accordance with the present invention to incorporate equivalent amount of Methylcobalamin and Cyanocobalamin respectively, to accurately compare the bioavailability of the two formulations. The pH of Nascobal was found to be 5 and therefore the pH of the Cyanocobalamin formulation of the present invention (Test G) was adjusted to about 4.96.The results of the bio-equivalence study are as shown below, also in Figure 6 and Figure 7. The pH of the methylcobalamin formulation was adjusted to 6.25 ± 0.25.

**Table 8**

| **Test** | **Component Measured** | **AUC0-t (pg.hr/ml) (Baseline corrected)** |
|---|---|---|
| Test F | Methylcobalamin | 43376.84 |
| | Total B12 | 47913.54 |
| Test G | Methylcobalamin | -3314.79 |
| | Total B12 | 67567.87 |
| Nascobal | Methylcobalamin | 461.5 |
| | Total B12 | 30022.68 |

It was most surprisingly found that Cyanocobalamin formulation containing sodium glycocholate prepared as per the present invention produced more than double the bioavailability of Total Vitamin B12 as compared to the marketed Cyanocobalamin spray (Nascobal). Moreover, the total vitamin B12 levels of Methylcobalamin formulation of present invention (Test F) were significantly higher as compared to Nascobal.

It was also found that Methylcobalamin formulation of present invention (Test F) produced significantly higher levels of Methylcobalamin as compared to other two Cyanocobalamin formulations. It is pertinent to note only the formulation of Methylcobalamin as per the present invention provided high levels of Methylcobalamin which is the active Co-enzyme of Vitamin B12.

The results unambiguously establish that the intra-nasal formulation comprising Cyanocobalamin with at least one penetration enhancer, optionally preservative, chelating agent, humectants or a combination thereof with pH of about 4 to about 6 and viscosity of about 1-200 CPS (mPas) provides significantly increased systemic delivery of the drug resulting in enhanced effectiveness of the therapy without compromising on the patient compliance or the stability of the formulation. The invention is further exemplified with the following non-limiting examples.

### Example 1

| **Ingredients** | **Amounts** |
|---|---|
| Methylcobalamin | 500 mcg |
| Benzalkonium Chloride | 0.02 mg |
| Glycerin | 2.23 mg |
| Glycofurol | 1.00 mg |
| Sodium citrate di-hydrate | 0.32 mg |
| Citrate Acid Anhydrous (5%w/v) | q.s. |
| Purified water | q.s. to 0.1 ml |

The pH of the formulation was adjusted to 6.25 ± 0.5 and the viscosity of the formulation was found to be 1.28 CPS (mPas).

### Example 2

| **Ingredients** | **Amounts** |
|---|---|
| Methylcobalamin | 500mcg |
| Benzalkonium Chloride | 0.02 mg |
| Glycerin | 2.23 mg |
| Glycofurol | 1.00 mg |
| Sodium Glycocholate | 2.00 mg |
| Sodium citrate di-hydrate | 0.32 mg |
| Citrate Acid Anhydrous (5%w/v) | q.s. |
| Purified water | q.s. to 0.1 ml |
| pH Adjusted to | 6.28 |

The pH of the formulation was adjusted to 6.25 + 0.5 and the viscosity of the formulation was found to be 2.3 CPS (mPas).

### Example 3

| **Ingredients** | **Amounts** |
|---|---|
| Methylcobalamin | 500mcg |
| Benzalkonium Chloride | 0.02 mg |
| Glycerin | 2.23 mg |
| Glycofurol | 1.00 mg |
| Sodium Glycocholate | 1.00 mg |
| Sodium citrate di-hydrate | 0.32 mg |
| Citric Acid Anhydrous (5%w/v) | q.s. |
| Water | q.s. to 0.1 ml |

The pH of the formulation was adjusted to 6.25 ± 0.5 and the viscosity of the formulation was found to be 1.57 CPS (mPas).

### Example 4

| **Ingredients** | **Amounts** |
|---|---|
| Methylcobalamin | 500 mcg |
| Benzalkonium Chloride | 0.02 mg |
| Glycerin | 2.23 mg |
| Glycofurol | 1.00 mg |
| Sodium Glycocholate | 1.50 mg |
| Sodium citrate di-hydrate | 0.32 mg |
| Citric Acid Anhydrous (5%w/v) | q.s. |
| Water | q.s. to 0.1 ml |

The pH of the formulation was adjusted to 6.25 ± 0.5 and the viscosity of the formulation was found to be 2.0 CPS (mPas).

### Example 5

| **Ingredients** | **Amounts** |
|---|---|
| Methylcobalamin | 500 mcg |
| Benzalkonium Chloride | 0.02 mg |
| Glycerin | 2.23 mg |
| Glycofurol | 1.00 mg |
| Sodium Glycocholate | 0.50 mg |
| Sodium citrate di-hydrate | 0.32 mg |
| Citric Acid Anhydrous (5%w/v) | q.s. |
| Water | q.s. to 0.1 ml |

The pH of the formulation was adjusted to 6.25 ± 0.5 and the viscosity of the formulation was found to be 1.43 CPS (mPas).

### Example 6

| **Ingredients** | **Amounts** |
|---|---|
| Methylcobalamin | 500 mcg |
| Benzalkonium Chloride | 0.02 mg |
| Glycerin | 5 mg |
| Glycofurol | 30 mg |
| Sodium citrate di-hydrate | 0.32 mg |
| Citric Acid Anhydrous (5%w/v) | q.s. |
| water | q.s. to 0.1 ml |

The pH of the formulation was adjusted to 6.25 ± 0.5 and the viscosity of the formulation was found to be 8 CPS (mPas).

### Example 7

| **Ingredients** | **Amounts** |
|---|---|
| Cyanocobalamin | 500 mcg |
| Benzalkonium Chloride | 0.02 mg |
| Glycerin | 2.23 mg |
| Glycofurol | 1.00 mg |
| Sodium Glycocholate | 1.00 mg |
| Sodium citrate di-hydrate | 0.32 mg |
| Citric Acid Anhydrous (5%w/v) | q.s. |
| Water | q.s. to 0.1 ml |

The pH of the formulation was adjusted to 4.5 ± 0.5 as per the marketed formulation of Cyanocobalamin. The viscosity of the formulation was observed to be 1.6 CPS (mPas).

### Example 8

| **Ingredients** | **Amounts** |
|---|---|
| Methylcobalamin | 500 mcg |
| Benzalkonium Chloride | 0.02 mg |
| Glycerin | 2.23 mg |
| Glycofurol | 1.00 mg |
| Carbopol 934P | 0.30 mg |
| Sodium citrate di-hydrate | 0.32 mg |
| NaOH solution (1%w/v) | q.s. |
| Water | q.s. to 0.1 ml |

The pH of the formulation was adjusted to 6.25 ± 0.5 and the viscosity of the formulation was observed to be 10 CPS (mPas).

### Example 9

| **Ingredients** | **Amounts** |
|---|---|
| Methylcobalamin | 500 mcg |
| Benzalkonium Chloride | 0.02 mg |
| Glycerin | 5 mg |
| Propylene Glycol | 10 mg |
| Sodium citrate di-hydrate | 0.32 mg |
| Citric Acid Anhydrous (5%w/v) | q.s. |
| Water | q.s. to 0.1 ml |

The pH of the formulation was adjusted to 6.25 ± 0.5 and the viscosity of the formulation was observed to be in the range as per the present invention

### Example 10

| **Ingredients** | **Amounts** |
|---|---|
| Methylcobalamin | 500 mcg |
| Benzalkonium Chloride | 0.02 mg |
| Glycerin | 5 mg |
| Polyethylene Glycol | 15 mg |
| Sodium citrate di-hydrate | 0.32 mg |
| Citric Acid Anhydrous (5%w/v) | q.s. |
| Water | q.s. to 0.1 ml |

The pH of the formulation was adjusted to 6.25 ± 0.5 and the viscosity of the formulation was observed to be in the range as per the present invention.

Compositions of substantially higher viscosities can be prepared when the level of carbopol, Polyethylene glycol is increased.

## Claims

1. A stable intranasal aqueous composition comprising methylcobalamin in concentration from 500mcg/0.1ml to 1500mcg/0.1ml, co-solvents/solubilisers or mixtures thereof in water, wherein the cosolvent/solubiliser is selected from glycofurol, propylene glycol, polyethylene glycols, or mixtures thereof, optionally a penetration enhancer, and wherein the pH of the composition is 6 to 6.5 and viscosity of 1 to 200 mPas.

2. A stable intranasal aqueous composition comprising cyanocobalamin in concentration from 500mcg/0.1ml to 1500mcg/0.1ml, co-solvents/solubilisers or mixtures thereof in water, and at least one penetration enhancer, wherein the cosolvent/solubiliser is selected from glycofurol, propylene glycol, polyethylene glycols, or mixtures thereof, wherein the penetration enhancer is selected from bile salts such as sodium glycocholate and wherein the pH of the composition is 4 to 6 and viscosity of 1 to 200 mPas.

3. A stable intranasal composition as claimed in any of the preceding claims, wherein the co-solvents/solubilisers are in the range of 1 to 35% w/v.

4. A stable intranasal composition as claimed in claims 1 and 2, wherein the amount of penetration enhancer is up to 2.5 % w/v of the composition.

## Patentansprüche

1. Stabile intranasale wässrige Zusammensetzung, umfassend Methylcobalamin in einer Konzentration von 500 µg / 0,1 ml bis 1500 µg / 0,1 ml, Co-Lösungsmittel / Lösungsvermittler oder Gemische davon in Wasser, wobei das Co-Lösungsmittel / der Lösungsvermittler ausgewählt ist aus Glycofurol, Propylenglycol, Polyethylenglycolen oder Gemischen davon, optional einem Penetrationsverstärker, und wobei der pH der Zusammensetzung 6 bis 6,5 beträgt und die Viskosität 1 bis 200 mPas beträgt.

2. Stabile intranasale wässrige Zusammensetzung, umfassend Cyanocobalamin in einer Konzentration von 500 µg / 0,1 ml bis 1500 µg / 0,1 ml, Co-Lösungsmittel / Lösungsvermittler oder Gemische davon in Wasser und mindestens einen Penetrationsverstärker, wobei das Co-Lösungsmittel / der Lösungsvermittler ausgewählt ist aus Glycofurol, Propylenglycol, Polyethylenglycolen oder Gemischen davon, wobei der Penetrationsverstärker ausgewählt ist aus Gallensalzen, wie etwa Natriumglycocholat, und wobei der pH der Zusammensetzung 4 bis 6 beträgt und die Viskosität 1 bis 200 mPas beträgt.

3. Stabile intranasale Zusammensetzung nach einem der vorhergehenden Ansprüche, wobei die Co-Lösungsmittel / Lösungsvermittler im Bereich von 1 bis 35 % Gew./Vol. sind.

4. Stabile intranasale Zusammensetzung nach den Ansprüchen 1 und 2, wobei die Menge an Penetrationsverstärkern bis zu 2,5 % Gew./Vol. der Zusammensetzung beträgt.

## Revendications

1. Composition aqueuse intranasale stable comprenant de la méthylcobalamine dans une concentration de 500 mcg/ 0,1 ml à 1500 mcg/0,1 ml, des cosolvants/solubilisants ou des mélanges de ceux-ci dans de l'eau, dans laquelle le cosolvant/solubilisant est choisi parmi le glycofurol, le propylène glycol, les polyéthylènes glycols, ou des mélanges de ceux-ci, éventuellement un agent amplifiant la pénétration, et dans laquelle le pH de la composition est de 6 à 6,5 et la viscosité de 1 à 200 mPa.s.

2. Composition aqueuse intranasale stable comprenant de la cyanocobalamine dans une concentration de 500 mcg/ 0,1 ml à 1500 mcg/0,1 ml, des cosolvants/solubilisants ou des mélanges de ceux-ci dans de l'eau, et au moins un agent amplifiant la pénétration, dans laquelle le cosolvant/solubilisant est choisi parmi le glycofurol, le propylène glycol, les polyéthylènes glycols, ou des mélanges de ceux-ci, dans laquelle l'agent amplifiant la pénétration est choisi parmi des sels biliaires tels que le glycocholate de sodium et dans laquelle le pH de la composition est de 4 à 6 et la viscosité de 1 à 200 mPa.s.

3. Composition intranasale stable selon l'une quelconque des revendications précédentes, dans laquelle les cosolvants/solubilisants se situent dans la plage de 1 à 35 % p/v.

4. Composition intranasale stable selon les revendications 1 et 2, dans laquelle la quantité d'agent amplifiant la pénétration représente jusqu'à 2,5 % p/v de la composition.
